# EUROPÄISCHE PATENTSCHRIFT

(11) **EP 1 510 227 B1**
(45) Veröffentlichungstag und Bekanntmachung des Hinweises auf die Patenterteilung: **05.10.2011**
(21) Anmeldenummer: 04019869.9
(22) Anmeldetag: 21.08.2004
(51) Int. Cl.: A61L 2/26, B08B 3/04, A61L 2/07, B67B 1/03

(54) **Behandlungs- und Transportbehälter**
Treatment and transport container
Conteneur de traitement et de transport

(30) Priorität: 01.09.2003 DE 10340169; 29.04.2004 DE 202004006896 U
(43) Veröffentlichungstag der Anmeldung: 02.03.2005
(73) Patentinhaber: Atec Pharmatechnik Gmbh, 24966 Sörup (DE)
(72) Erfinder: Mumm, Hans-Werner, 24966 Sörup (DE)
(74) Vertreter: Thomas, Götz

(56) Entgegenhaltungen:
- EP-A- 0 418 450
- EP-A- 0 586 307
- WO-A-00/74735
- DE-A- 2 739 169
- DE-A- 4 409 659
- DE-A- 19 916 720
- DE-C- 969 308

## Beschreibung

Die Erfindung betrifft einen Behandlungs- und Transportbehälter zur Behandlung und zum Transport von kleineren Gegenständen, wie Teilen von Spritzen, Verschlusstopfen von Infusionsflaschen, Vials für pharmazeutische Zwecke oder dergleichen, insbesondere zur Reinigung und Sterilisation der Gegenstände, mit einem Unterteil, der einen in Richtung einer Behälterlängsachse im Wesentlichen gleichbleibenden Innenquerschnitt aufweist, einem an einem Stirnende des Unterteils angebrachten Bodenteil, der eine mit einem Absperrorgan versehene verschließbare Öffnung für ein Behandlungsmedium aufweist, sowie einem am entgegengesetzten Stirnende des Unterteils angebrachten verjüngten Oberteil, der eine mit einem Absperrorgan versehene verschließbare Befüll- und Entleeröffnung aufweist, die in einer Befüllstellung des Behälters nach oben und in einer Entleerstellung des Behälters nach unten weist, wobei einander gegenüberliegende Wandabschnitte des Oberteils unter einem Öffnungswinkel (α) von weniger als 90 Grad von der Befüll- und Entleeröffnung weg divergieren.

Behälter dieser Art sind zum Beispiel aus der DE 199 16 720 A1 oder aus der DE 44 09 659 A1 bekannt. Ein weiterer derartiger Behälter ist in der DE 103 07 444 A1 der Anmelderin beschrieben. Diese Behälter werden insbesondere in einer Reinraumumgebung eingesetzt, um ein aus Spritzenteilen, Verschlussstopfen aus Gummi oder Kunststoff für Infusionsflaschen oder Vials und dergleichen bestehendes Gut nach dem Einfüllen in den Behälter im Behälterinneren zu reinigen, zu sterilisieren und ggf. zu silikonisieren. Der befüllte Behälter kann dazu an einer schwenkbaren Halterung einer Reinigungsvorrichtung aufgehängt werden, die mit Anschlüssen zum Andocken der Befüll- und Entleeröffnung sowie der Öffnung im Boden des Behälters versehen ist, so dass nach dem Andocken und dem Öffnen der Absperrorgane ein oder mehrere flüssige oder dampfförmige Behandlungs- oder Prozessmedien, wie beispielsweise heißes Wasser oder Heißdampf, unter gleichzeitigem Verschwenken des Behälters durch diesen hindurchgeleitet werden können, um das darin enthaltene Gut der gewünschten Behandlung zu unterziehen und zugleich in Bewegung zu versetzen. Nach der Behandlung wird der Behälter abgenommen und zum Beispiel zu einer Übergabestation transportiert, wo er in seiner Entleerstellung, d.h. in umgedrehtem Zustand, angedockt wird, so dass das behandelte Gut nach dem Öffnen des Absperrorgans von selbst durch die nach unten weisende Befüll- und Entleeröffnung herausfällt.

Die bekannten Behälter weisen einen zylindrischen Unterteil, einen den Unterteil in der Befüllstellung nach unten verschließenden Bodenteil und einen kegelstumpfförmigen Oberteil mit einer kreisförmigen Befüll- und Entleeröffnung auf, deren Mittelpunkt auf der Behälterlängsachse liegt. Um in der Entleerstellung eine schnelle Entleerung zu gewährleisten, sollte der in einer Längsschnittebene des Behälters von gegenüberliegenden divergierenden Mantellinien des Oberteils eingeschlossene Öffnungswinkel höchstens 90 Grad betragen. Vorzugsweise werden Öffnungswinkel von 60 Grad verwendet, was jedoch bei der Behandlung von Verschlussstopfen aus Gummi nicht ausreichend ist, um sicher zu verhindern, dass in der Entleerstellung einige Verschlusstopfen unmittelbar oberhalb des Übergangs zwischen dem nach oben weisenden zylindrischen Unterteil und dem nach unten weisenden trichterförmigen Oberteil an der Behälterwand hängen bleiben. Eine weitere Verkleinerung des Öffnungswinkels führt jedoch bei gleichbleibendem Durchmesser des Unterteils zu einer noch weiteren Vergrößerung der Höhe des Behälters, die bereits bei Öffnungswinkeln von 60 Grad in beengten Räumen Probleme bereiten kann.

Weitere Probleme bereitet die Tatsache, dass die Außenseite der Befüll- und Entleeröffnung bzw. des daran angebrachten Absperrorgans nach einem Transport des Behälters mit dem sterilisierten Gut durch eine kontaminierte Umgebung erneut sterilisiert werden muss, bevor der Behälter zur Entleerung an einer Übergabestation angedockt werden kann.

Weiter ist aus der DE-A-27 39 169 bereits ein Behälter bekannt, der eine in einem seitlichen Abstand von der Behälterlängsachse angeordnete Befüllöffnung aufweist. Der bekannte Behälter wird jedoch beim Entleeren nicht umgedreht, sondern behält seine Ausrichtung bei, weshalb er in seinem Unterteil mit einer zusätzlichen, in der Befüll- und Entleerstellung nach unten weisenden Entleeröffnung versehen werden muss.

Die DE-C-969308 offenbart einem Behälter für Zement, Kalksteim oder andere pulverförmige Stoffe, wobei die Auslaßöffnung in einem seitlichen Abstand von der Längsachse des Behälters angeordnet ist.

Ausgehend hiervon liegt der Erfindung die Aufgabe zugrunde, einen Behälter der eingangs genannten Art dahingehend zu verbessern, dass die Höhe des Behälters bei gleichbleibendem Behälterquerschnitt und Prozessvolumen verringert werden kann, ohne dass die Gefahr besteht, dass beim Entleeren Gut im Oberteil oder am Übergang zwischen Ober- und Unterteil hängen bleibt. Eine weitere Aufgabe der Erfindung ist es, nach einem Transport des Behälters durch eine unsterile Umgebung vor einer Entleerung des Behälters eine erneute Sterilisierung der Außenseite seiner Befüll- und Entleeröffnung zu vermeiden.

Diese Aufgabe wird erfindungsgemäß mit den Merkmalen des Anspruchs 1 gelöst.

Der Erfindung liegt die überraschende Erkenntnis zugrunde, dass sich bei gleichbleibendem Querschnitt des Unterteils und damit gleichbleibendem Prozessvolumen sowie bei gleichbleibendem Öffnungswinkel des Oberteils die Bauhöhe des Behälters umso mehr verringert, je weiter man die Befüll- und Entleeröffnung von der Behälterlängsachse weg verlagert.

Bei einer solchen Verlagerung verändert sich gleichzeitig in einer zur Richtung der Verlagerung parallelen Schnittebene des Behälters der Übergangswinkel zwischen aneinandergrenzenden Wandabschnitten des Unterteils und des Oberteils, wobei er auf der einen Seite kleiner und gleichzeitig auf der anderen, diametral entgegengesetzten Seite größer wird. Diese Tatsache kann ausgenutzt werden, um das Hängenbleiben von Gut an der Behälterwand zu verhindern, indem man dafür sorgt, dass das Gut beim Entleeren des Behälters über die Seite mit dem größeren Übergangswinkel zur Befüll- und Entleeröffnung rutscht.

Auf dieser Seite wird darüber hinaus auch der Abstand zwischen dem oberen Rand des Unterteils und der Öffnung und damit auch der Bewegungsweg des Gutes beim Entleeren verkürzt, wodurch wiederum die Entleerung beschleunigt wird. Durch die geringere Gutbewegung beim Entleeren wird zudem die gegenseitige Reibung vermindert, so dass weniger Abrieb am Gut entstehen kann. Ein Gutaustrag über die Seite mit dem größeren Übergangswinkel wird erreicht, indem der Behälter gemäß einer weiteren vorteilhaften Ausgestaltung der Erfindung über diese Seite aus der Befüllstellung in die Entleerstellung geschwenkt wird, wobei darüber hinaus der Schwenkwinkel auf weniger als 180 Grad verkleinert und damit die zum Verschwenken benötigte Zeit verkürzt werden kann.

Eine andere bevorzugte Ausgestaltung der Erfindung sieht vor, dass zumindest auf der Innenseite des Oberteils jeder Punkt auf einem umlaufenden Rand der Befüll- und Entleeröffnung durch eine gerade Mantellinie mit einem benachbarten Punkt auf einem oberen Rand des Unterteils verbunden ist, wobei die Steigung dieser Mantellinien in Bezug zu einer vom oberen Rand des Unterteils aufgespannten Ebene infolge des seitlichen Versatzes der Befüll- und Entleeröffnung um den Umfang des Oberteils herum zwischen einem Maximum und einem diametral entgegengesetzten Minimum variiert.

Wie bereits angegeben, wird die Bauhöhe des Behälters umso kleiner, je weiter die Befüll- und Entleeröffnung aus der Behälterlängsachse heraus verlagert wird. Eine minimale Bauhöhe wird erreicht, wenn der umlaufende Rand der Befüll- und Entleeröffnung an mindestens einer Stelle mit einem benachbarten Wandabschnitt des Unterteils fluchtet, der Übergangswinkel also 180 Grad beträgt. Dadurch wird auch die Gefahr eines Hängenbleibens von Gut minimiert, da die aneinandergrenzenden Wandabschnitte des Unterteils und des Oberteils sowie der Rand der Befüll- und Entleeröffnung an dieser Stelle des Behälterumfangs auf einer zur Behälterlängsachse parallelen Geraden liegen und somit keine das Hängenbleiben begünstigenden Ecken oder Winkel vorhanden sind. Bei dieser Anordnung der Befüll- und Entleeröffnung schneidet die Behälterlängsachse eine von einem Rand der Befüll- und Entleeröffnung aufgespannte Ebene unter einem Schnittwinkel, der vorzugsweise gleich dem Öffnungswinkel ist.

Gemäß einer weiteren bevorzugte Ausgestaltung der Erfindung weist der Unterteil einen kreisförmigen Querschnitt auf, während der Oberteil in etwa die Form eines Kegelstumpfs mit schräger Grundfläche besitzt. Alternativ könnte jedoch der Unterteil einen polygonalen bzw. elliptischen Querschnitt und der Oberteil die Form einer entsprechenden mehrseitigen Pyramide bzw. eines schrägen Kegelstumpfs besitzen.

Durch die Verlagerung der Befüll- und Entleeröffnung aus der Behälterlängsachse heraus wird darüber hinaus in Verlängerung der Behälterlängsachse ein geschlossener, schwach geneigter Wandabschnitt auf dem Oberteil gebildet, der genutzt werden kann, um dort eine zusätzliche Öffnung zur Zufuhr bzw. Abfuhr von Behandlungsmedium vorzusehen, so dass die Befüll- und Entleeröffnung während der Behandlung verschlossen bleiben kann. Dies wiederum erlaubt es, die Befüll- und Entleeröffnung mit einem integrierten Deckel für einen sogenannten Rapid-Transfer-Port zu versehen, der die Möglichkeit einer Übergabe des sterilisierten Guts aus dem Behälter an die Übergabestation ohne eine vorangehende Sterilisation der Andockstelle bietet.

Rapid-Transfer-Ports sind Doppelklappensysteme, die bereits seit langem zum Beispiel von der Fa. SNE La Calhene, Frankreich, unter der Bezeichnung DPTE-System vertrieben werden. Diese Rapid-Transfer-Ports umfassen einen an der Übergabestation angeordneten sogenannten Alpha-Teil, bestehend aus einer Klappe zum Andocken der Behälteröffnung, einem die Klappe umgebenden Flansch und einer zwischen Flansch und Klappe angeordneten Mehrfach-Ringdichtung, sowie einen am Behälter angeordneten Beta-Teil, bestehend aus einer die Behälteröffnung verschließenden Klappe, einem die Klappe umgebenden Flansch und einer weiteren Mehrfach-Ringdichtung. Beim Andocken des Beta-Teils des Behälters an den Alpha-Teil der Übergabestation werden die einander zugewandten Außenseiten der Klappen radial nach außen zu durch die Ringdichtung des Alpha-Teils abgedichtet, die sich dichtend gegen der Rand der Klappe des Behälters anlegt. Diese kann dann zusammen mit der Klappe der Übergabestation nach einer Seite aufgeschwenkt werden, um die sterile Umgebung im Behälter und in der Übergabestation zu verbinden, ohne dass die Gefahr einer Kontamination besteht.

Ein derartiger Rapid-Transfer-Port ist in der WO 00/74735 A1 auch bereits in Verbindung mit einem Behälter der eingangs genannten Art zur Sterilisierung von pharmazeutischem Gut beschrieben worden, wobei der Deckel für den Rapid-Transfer-Port dort jedoch auf einem zusätzlichen, als separates Bauteil ausgebildeten Y-förmigen Verbinder angeordnet ist, der die Bauhöhe des Behälters weiter vergrößert.

Gemäß der Erfindung weist der Behälter selbst im Bereich der Befüll- und Entleeröffnung einen integrierten, außerhalb des Absperrventils angeordneten Beta-Teil eines Rapid-Transfer-Ports auf, der beim Ankoppeln des Behälters an die Übergabestation mit dem dort angebrachten Alpha-Teil koppelbar ist, so dass die einander zugewandten Außenseiten der beiden Deckel gas- und flüssigkeitsdicht zwischen den Deckeln eingeschlossen werden. Das Absperrventil verhindert bei der Behandlung eine Belastung des Beta-Teils infolge des Drucks im Behälter sowie in der Entleerstellung durch das Gewicht der Gegenstände und ist vorzugsweise als Dosierventil ausgebildet, das in der Entleerstellung eine dosierte Abgabe der Gegenstände durch den zuvor geöffneten Rapid-Transfer-Port hindurch gestattet.

Die zusätzliche Öffnung zur Zufuhr bzw. Abfuhr von Behandlungsmedium ist vorzugsweise als Anschlussstutzen ausgebildet, der auf die Mantelfläche des verjüngten Oberteils aufgesetzt ist. Der Anschlussstutzen ist mit einem Absperrorgan versehen, so dass er vor dem Abkoppeln des Behälters von der Reinigungsvorrichtung verschlossen werden kann, um während des Transports zur Übergabestation und bei der Entleerung in dieselbe die Sterilität im Behälter aufrechtzuerhalten. Der Anschlussstutzen kann zweckmäßig als ein auf die Mantelfläche des Oberteils aufgesetzter und in Verlängerung der Mantelfläche durch ein Lochblech verschlossener Konus mit einer mit der Längsachse des Behälterunterteils fluchtenden Mittelachse ausgebildet sein, oder er kann bevorzugter in Bezug zur Behälterlängsachse an einer zur Befüll- und Entleeröffnung im Wesentlichen diametral entgegengesetzten Stelle angeordnet sein, so dass er einen möglichst großen Abstand und Höhenunterschied von dieser aufweist, wodurch ohne ein Lochblech verhindert werden kann, dass behandelte Gegenstände in den Anschlussstutzen fallen und darin zurückgehalten werden, wenn der Behälter zwecks Entleerung so in die Entleerstellung gedreht wird, dass sich die Befüll- und Entleeröffnung voran und der Anschlussstutzen hinterher bewegt.

Im folgenden wird die Erfindung anhand zweier in der Zeichnung dargestellter Ausführungsbeispiele näher erläutert. Es zeigen:
Fig. 1a: eine Querschnittsansicht eines erfindungsgemäßen Behandlungs- und Transportbehälters;
Fig. 1b: eine Querschnittsansicht eines entsprechenden Behandlungs- und Transportbehälters gemäß dem Stand der Technik zu Vergleichszwecken;
Fig. 2 und 3: Querschnittsansichten des Behandlungs- und Transportbehälters aus Fig. 1a in der Befüll- bzw. Entleerstellung;
Fig. 4: eine Abwicklung einer Innen- bzw. Außenwand des verjüngten Oberteils des Behälters aus Fig. 1a;
Fig. 5: eine Querschnittsansicht eines weiteren erfindungsgemäßen Behandlungs- und Transportbehälters.
Fig. 6: eine Querschnittsansicht eines noch weiteren erfindungsgemäßen Behandlungs- und Transportbehälters.

Der in der Zeichnung dargestellte erfindungsgemäße Behandlungs- und Transportbehälter 2 dient zur Reinigung, Sterilisation und ggf. zur Silikonisierung von Verschlussstopfen für pharmazeutische Verpackungsbehälter, sowie zum Transport der Verschlusstopfen vor und nach ihrer Reinigung und Sterilisation. Wegen der hohen Anforderungen an die Reinheit erfolgt die Behandlung der Stopfen ebenso wie das Befüllen und das Entleeren des Behälters 2 vorzugsweise innerhalb eines Reinraums. Während das Befüllen des Behälter 2 mit den unbehandelten Stopfen an einer Befüllstation erfolgt, wird die Reinigung und Sterilisation der Stopfen mittels einer Reinigungs- und Sterilisationsvorrichtung vorgenommen. Das Entleeren des Behälters erfolgt an einer Übergabestation, zum Beispiel einer Übergabestation einer Verpackungsmaschine, in der die Stopfen in sterilem Zustand einzeln auf zuvor gefüllten pharmazeutischen Verpackungsbehältern angebracht werden. Eine zur Reinigung, Sterilisation und Silikonisierung von Stopfen im Behälter 2 geeignete Reinigungs- und Sterilisationsvorrichtung ist in der eingangs genannten DE 103 07 444 A1 ausführlich beschrieben, deren Offenbarung zur Vermeidung einer Wiederholung hier aufgenommen werden soll.

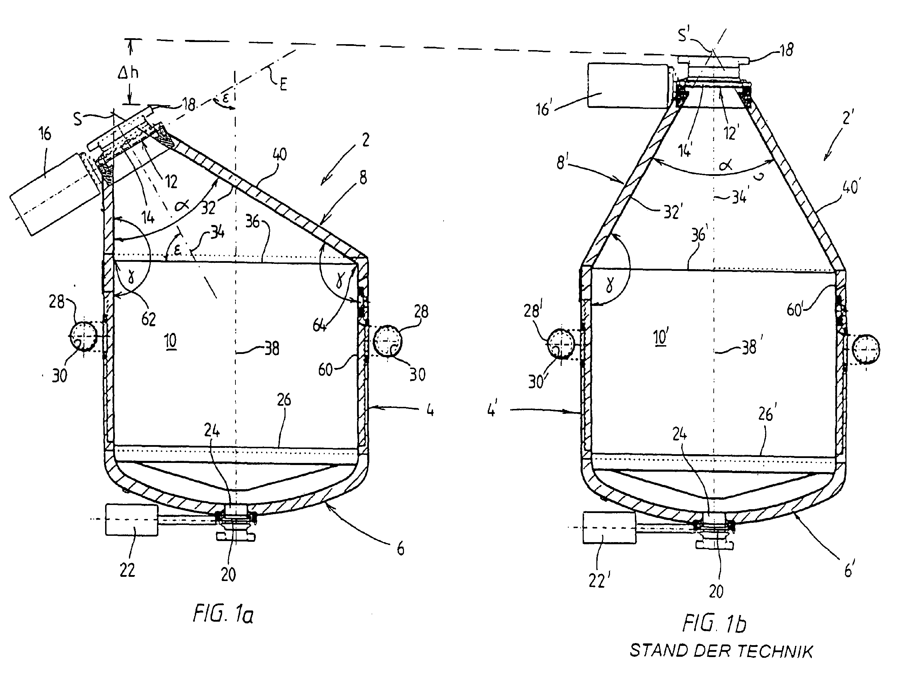

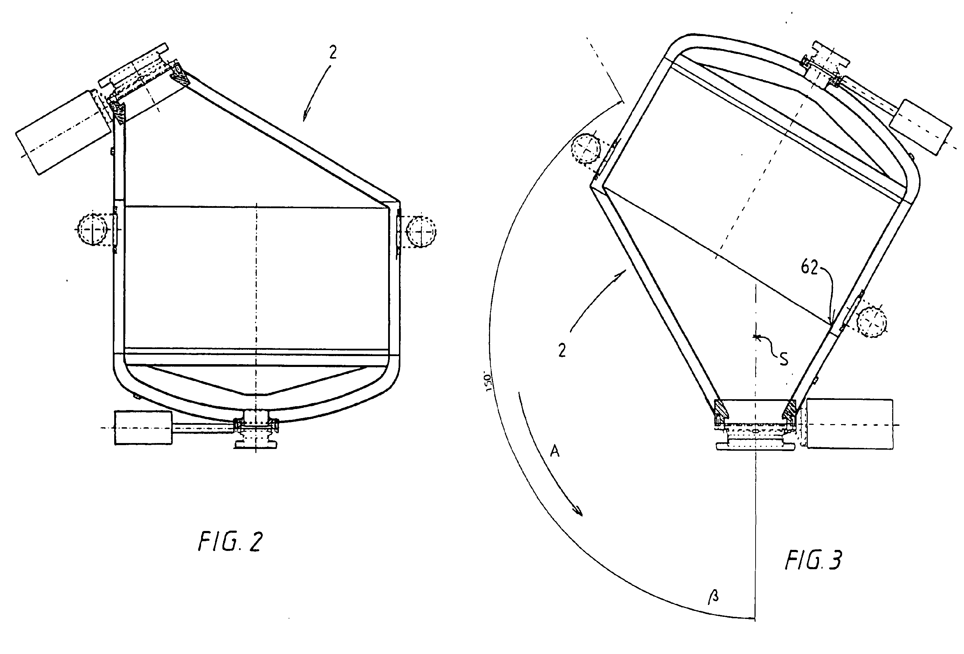

Wie am besten in Fig. 1a dargestellt, besteht der druckfeste doppelwandige Behandlungs- und Transportbehälter 2 ebenso wie ein zu Vergleichszwecken in Fig. 1b dargestellter konventioneller Behandlungs- und Transportbehälter 2' im wesentlichen aus einem zylindrischen Unterteil 4 bzw. 4', einem konvex nach unten gewölbten Bodenteil 6 bzw. 6' sowie einem nach oben zu verjüngten Oberteil 8 bzw. 8', die miteinander verschweißt sind und einen Behälterinnenraum 10 bzw. 10' flüssigkeitsdicht umschließen. Am oberen Ende des Oberteils 8 bzw. 8' befindet sich eine Befüll- und Entleeröffnung 12 bzw. 12' mit kreisförmigem Öffnungsquerschnitt, die durch ein Absperrventil 14 bzw. 14' mit Motorantrieb 16 bzw. 16' verschließbar ist. Durch die Öffnung 12 bzw. 12' wird der Behälter 2 bzw. 2' in seiner in Fig. 1a bzw. 1b dargestellten aufrechten Befüllstellung mit den zu reinigenden und zu sterilisierenden Verschlüssen befüllt, während das Entleeren des Behälters 2 in der in Fig. 3 dargestellten Entleerstellung erfolgt, in der die Öffnung 12 nach unten weist. Bei der Entleerung fallen die gereinigten und sterilisierten Verschlüsse durch ihre Schwerkraft aus dem Behälter 2 bzw. 2' in die Übergabestation, die einen zum einem Öffnungsflansch 18 bzw. 18' des Behälters 2 bzw. 2' passenden Andockmechanismus aufweist.

Am tiefsten Punkt des Bodenteils 6 bzw. 6' befindet sich eine weitere durch ein Absperrventil 20 bzw. 20' mit Motorantrieb 22 bzw. 22' verschließbare Öffnung 24 bzw. 24', durch die nach dem Ankoppeln des Behälters 2 bzw. 2' an die Reinigungs- und Sterilisationsvorrichtung ebenso wie durch die Befüll- und Entleeröffnung 12 bzw. 12' Heißwasser, Kaltwasser, ein Wasser-Luft-Gemisch, Trocknungsluft, Dampf oder andere flüssige oder gasförmige Behandlungsmedien in den Behälter 2 bzw. 2' zugeführt oder aus diesem abgeführt werden können. Im Inneren ist der Behälter 2 bzw. 2' in einem geringen Abstand über dem Bodenteil 6 bzw. 6' mit einem siebförmigen Zwischenboden 26 bzw. 26' versehen, der bei der Behandlung einen Hindurchtritt des Behandlungsmediums gestattet und bei aufrecht stehendem Behälter 2 bzw. 2' ein Abtropfen der Stopfen erlaubt.

Der Behälter 2 bzw. 2' weist weiter zwei diametral entgegengesetzte seitliche Ausleger 28 bzw. 28' mit zylindrischen Aufnahmebuchsen 30 bzw. 30' auf, die bei aufrechtem Behälter 2 bzw. 2' parallel und horizontal ausgerichtet sind. Diese Aufnahmebuchsen 30 bzw. 30' dienen zur lösbaren Befestigung des Behälters 2 bzw. 2' an einem Tragelement der Reinigungs- und Sterilisationsvorrichtung, das mit zwei überstehenden, in die Aufnahmebuchsen 30 bzw. 30' einführbaren parallelen Tragdornen versehen und mittels eines Schwenkantriebs um eine horizontale Schwenkachse SA schwenkbar ist.

Während bei dem in Fig. 1b dargestellten konventionellen Behälter 2 die Innenwand 32' des Oberteils 16' die Form eines geraden Kegelstumpfs aufweist, dessen Kegelachse 34' durch die gedachte Spitze S' des Kegelstumpfs, den Mittelpunkt der kreisförmigen Befüll- und Entleeröffnung 12' und den Mittelpunkt einer vom oberen Rand 36' des Unterteils 4' begrenzten Kreisfläche am Übergang zum Oberteil 8' verläuft und mit einer Längsachse 38' des Behälters 2' bzw. des Unterteils 8' fluchtet, weist bei dem erfindungsgemäßen Behälter 2 in Fig. 1a die Innenwand 32 (und auch die Außenwand 40) des Oberteils 8 im Wesentlichen ebenfalls die Form eines Kegelstumpfs auf, jedoch eines schrägen Kegelstumpfs, dessen Kegelachse 34 durch die gedachte Spitze S des Kegelstumpfs und die Mitte der kreisförmigen Befüll- und Entleeröffnung 12 verläuft, die vom oberen Rand 36 des Unterteils 4 begrenzte Kreisfläche aber außerhalb ihrer Mitte unter einem Schnittwinkel ε von 60 Grad schneidet. Umgekehrt schneidet die Behälterlängsachse 38 eine vom kreisförmigen Rand der Befüll- und Entleeröffnung 12 aufgespannte Ebene E unter einem Schnittwinkel ε von 60 Grad.

Die Innen- und die Außenwand 32 und 40 bzw. 32' und 40' bestehen jeweils aus Edelstahl und umschließen ein Isoliermaterial.

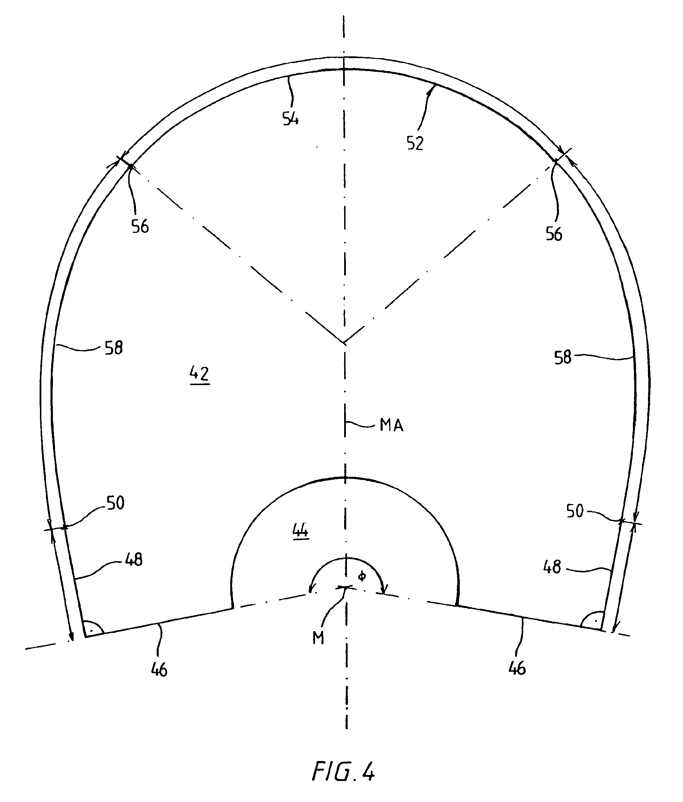

Wie man aus der in Fig. 4 dargestellten Draufsicht auf einen Zuschnitt 42 der Innen- oder Außenwand 32 oder 40 des Oberteils 8 des erfindungsgemäßen Behälters 2 sehen kann, wird der zu einer Mittelachse MA spiegelsymmetrische Zuschnitt 42 an einer Seite durch eine randoffene Aussparung 44 in Form eines Kreissektors und zwei durch den Mittelpunkt M des Kreissektors verlaufende Radienverlängerungen 46 begrenzt, die sich im Mittelpunkt M des Kreisausschnitts unter einem Sektorwinkel φ von 200 Grad schneiden. An die beiden Radienverlängerungen 46 schließen sich außen zwei zu den Radienverlängerungen 46 senkrechte, im Wesentlichen geradlinige Randabschnitte 48 an, die eine geringfügig kürzere Länge als die Radienabschnitte 46 aufweisen. Die Endpunkte 50 dieser beiden Abschnitte 48 sind durch einen konvexen Bogen 52 verbunden, dessen Mittelteil 54 zwischen den Punkten 56 im Wesentlichen kreisbogenförmig ist, während der Krümmungsradius seiner beiden Endteile 58 vom Mittelteil 54 aus bis zu den Endpunkten 50 der geradlinigen Randabschnitte 48 allmählich abnimmt.

Wenn bei einem beliebigen Zuschnitt, der wie der Zuschnitt 42 einseitig durch einen Kreissektor und zwei Radienverlängerungen des Kreissektors begrenzt wird, die beiden Radienverlängerungen zusammengeführt und übereinanderliegend zur Deckung gebracht werden, entsteht bekanntlich ein Kegelstumpf mit einer vom Rand des Kreisausschnitts begrenzten kreisförmigen Öffnung am verjüngten oberen Ende und einer durch die Spitze des gedachten Kegelstumpfs und durch die Mitte der Öffnung verlaufenden Kegelachse. Wenn dieser Kegelstumpf im Abstand von seinem oberen Ende entlang einer zur Kegelachse 34' senkrechten Ebene geschnitten wird, wie zum Beispiel die Wand 32' des Oberteils 8' des in Fig. 1b dargestellten konventionellen Behälters 2', weist seine Grundfläche eine kreisförmige Gestalt auf, deren Radius durch geeignete Wahl der Länge der Radienverlängerungen und des von diesen eingeschlossenen Winkels mit dem Radius des oberen Randes 36' der Innenwand 60' des Unterteils 4' zur Übereinstimmung gebracht werden kann, um den Ober- und den Unterteil 8' und 4' entlang dieses Randes 36' miteinander zu verbinden. Wenn jedoch der Kegelstumpf, wie zum Beispiel die Innenwand 32 des Oberteils 8 des erfindungsgemäßen Behälters 2 in Fig. 1b, entlang einer zur Kegelachse 34 geneigten Ebene geschnitten wird, weist seine Grundfläche eine elliptische Gestalt auf, die sich nicht mit dem kreisförmigen, zur Behälterachse 38 konzentrischen oberen Rand 36 des Unterteils 4 zur Übereinstimmung bringen und mit diesem verbinden lässt.

Wenn man jedoch, wie bei dem erfindungsgemäßen Behälter, den unteren Rand der Innenwand 32 bzw. der Außenwand 40 des Oberteils 8 bei der Fertigung in Richtung der Hauptachse der elliptischen Grundfläche zusammendrückt, nimmt die Ellipse allmählich eine kreisförmige Gestalt an, jedoch liegt dann der untere Rand der Wände 32 bzw. 40 nicht mehr in einer Ebene, so dass er sich ebenfalls nicht ohne weiteres mit dem ebenen oberen Rand 36 des Unterteils 4' verbinden lässt.

Bei dem erfindungsgemäßen Behälter ist daher der Zuschnitt 42 entlang der Randabschnitte 48, 58, 54, 58, 48 so angepasst worden, dass der beim Zusammenführen der Radienverlängerungen 46 gebildete Kegelstumpf mit einer zur Kegelachse 34 geneigten elliptischen Grundfläche nach einem Zusammendrücken der Grundfläche in Richtung der Hauptachse der Ellipse eine kreisförmige und in einer Ebene liegende Grundfläche aufweist, die sich anschließend problemlos mit dem ebenen kreisförmigen oberen Rand 36 des Unterteils 4 verbinden lässt.

Die genaue Form des Zuschnitts 42 erhält man dadurch, dass man die räumliche Lage des kreisförmigen Randes der Befüll- und Entleeröffnung 12 und des kreisförmigen oberen Randes 36 des Unterteils 4 vorgibt und diese beiden Ränder dann durch aneinandergrenzende finite Elemente in Form von schmalen Streifen verbindet, die anschließend unter Bildung des Zuschnitts 42 in einer Ebene aneinandergefügt werden.

Wie am besten in Fig. 1a bzw. 1b dargestellt, weist die Innenwand 32 sowohl bei dem Oberteil 8' des herkömmlichen Behälters 2' als auch bei dem Oberteil 8 des erfindungsgemäßen Behälters 2 einen Öffnungswinkel α von weniger als 90 Grad auf, wobei der Öffnungswinkel α bei den dargestellten Behältern 2, 2' 60 Grad beträgt. Als Öffnungswinkel α wird der Winkel zwischen zwei gegenüberliegenden Mantellinien der Innenwand 32 bzw. 32' in den Schnittebenen des Behälters 2, 2' bezeichnet, durch welche die Kegelachse 34 bzw. 34' verläuft. Wie man in Fig. 1a und 1b sieht, ist die Höhe des erfindungsgemäßen Behälters 2 bei gleichem Volumen des Unterteils 4 bzw. 4' und gleichem Öffnungswinkel α nicht unerheblich geringer als diejenige des herkömmlichen Behälters 2'.

Im Unterschied zum herkömmlichen Behälter, wo ein Übergangswinkel γ zwischen der Innenwand 60' des Unterteils 4' und der Innenwand 40' des Oberteils 8' am Übergang 36' um den gesamten Behälterumfang herum gleich bleibt und 150 Grad beträgt, verändert sich bei dem erfindungsgemäßen Behälter der Übergangswinkel γ am Übergang 36 um den Behälterumfang herum, wobei er an einer Stelle, in Fig. 1a bei 62, ein Maximum von 180 Grad besitzt, so dass die dort aneinandergrenzenden Wandabschnitte des Unterteils 4 und des Oberteils 8 miteinander fluchten, während er an einer diametral gegenüberliegenden Stelle, in Fig. 1a bei 64, ein Minimum von 120 Grad besitzt,

Die Befüll- und Entleeröffnung 12 des Behälters 2 ist in Bezug zu den Aufnahmebuchsen 28 so angeordnet, dass die fluchtenden Wandabschnitte des Unterteils 4 und des Oberteils 8 in der Nähe von einer der beiden Aufnahmebuchsen 28 liegen. Die Schwenkachse SA des Behälters 2 ist parallel zu den Längsachsen der zylindrischen Aufnahmebuchsen 28 und liegt in der Mitte zwischen diesen. Bei dem in Fig. 2 und 3 dargestellten Behälter ist die vom Rand der Befüll- und Entleeröffnung 12 aufgespannte Ebene E in der Entleerstellung horizontal ausgerichtet. Wenn der Behälter 2 zum Entleeren in Richtung des Pfeils A in Fig. 3 aus der Befüllstellung in die Entleerstellung geschwenkt wird, rutschen die im Behälterinneren 10 befindlichen Stopfen bei 62 in Bereich der fluchtenden Wandabschnitte des Unter- und Oberteils 4, 8 über den Übergang 36, wobei im Unterschied zu dem Behälter 2' dort keine Stopfen mehr hängen bleiben. Der Schwenkwinkel β zum Verschwenken des Behälters 2 aus der Befüllstellung in die Entleerstellung in Richtung des Pfeils A beträgt 150 Grad. Grundsätzlich könnte die zuletzt beschriebene Wirkung auch schon erzielt werden, wenn die bei 62 aneinandergrenzenden Wandabschnitte des Unter- und des Oberteils 4, 8 an dieser Stelle einen stumpfen Winkel einschließen, der jedoch vorzugsweise 160 Grad und am besten 170 Grad nicht unterschreiten sollte.

Eine ähnliche Wirkung wie bei dem zuvor beschriebenen Behälter 2 mit zylindrischem Unterteil 4 lässt sich erzielen, wenn der Unterteil des Behälters einen polygonalen, zum Beispiel quadratischen Querschnitt aufweist, und der Oberteil in Form eines polygonalen, zum Beispiel quadratischen Pyramidenstumpfs ausgebildet wird, dessen gedachte Pyramidenspitze im seitlichen Abstand von der Behälterlängsachse angeordnet ist. In diesem Fall besitzt natürlich auch die Befüll- und Entleeröffnung einen entsprechenden polygonalen, vorzugsweise quadratischen Öffnungsquerschnitt. Auch in diesem Fall sollte eine der an die Befüll- und Entleeröffnung angrenzenden Mantelflächen des pyramidenstumpfförmigen Oberteils mit einem angrenzenden Wandabschnitt des polygonalen Unterteils einen Übergangswinkel von mindestens 160 Grad, besser 170 Grad und vorzugsweise 180 Grad einschließen, so dass das im Behälter behandelte Gut auch hier beim Entleeren des Behälters nicht am Übergang zwischen dem Unterteil und dem Oberteil hängen bleibt. Bei Verwendung polygonaler Behälterquerschnitte lässt sich der Oberteil leichter an den Unterteil anpassen, jedoch ist die Länge der zur Herstellung erforderlichen Schweißnähte länger, weshalb bei den aus Edelstahl bestehenden Behältern ein runder Querschnitt bevorzugt wird.

Alternativ kann darüber hinaus auch ein Behälter gefertigt werden, der ein Unterteil mit einem elliptischen Querschnitt aufweist, so dass sein oberer Rand gut mit der elliptischen Grundfläche eines kegelstumpfförmigen Oberteils mit einer zu einer Seite versetzten Befüll- und Entleeröffnung, wie oben beschrieben, zusammenpasst.

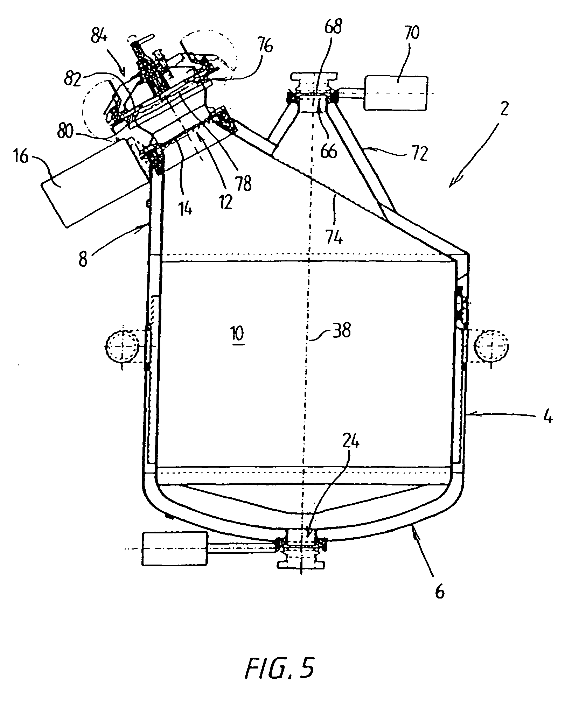

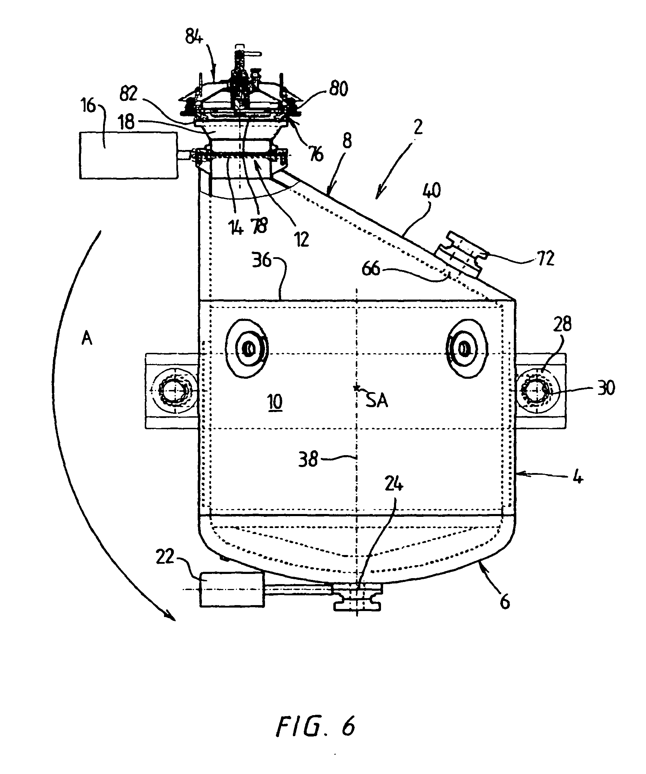

Die Figuren 5 und 6 zeigen zwei weitere Ausführungsformen eines erfindungsgemäßen Behandlungs- und Transportbehälters 2, deren Form im Wesentlichen derjenigen des zuvor beschriebenen Behälters 2 entspricht, bei dem jedoch eine zusätzliche Öffnung 66 vorgesehen ist, die gemeinsam mit der Öffnung 24 im Bodenteil 6 des Behälters 2 zur Zufuhr bzw. Abfuhr von Behandlungsmedien dient, so dass anders als bei dem Behälter 2 aus Fig. 1a, 2 und 3 die Befüll- und Entleeröffnung 12 nicht zu diesem zuletzt genannten Zweck verwendet werden braucht.

Wie die Öffnung 24 ist auch die Öffnung 66 durch ein Absperrventil mit Motorantrieb (in Fig. 6 nicht dargestellt) verschließbar, so dass sie vor dem Abkoppeln des Behälters 2 von der Reinigungs- und Sterilisationsvorrichtung verschlossen werden kann, um bei einem anschließenden Transport des Behälters 2 die Sterilität im Inneren desselben zu bewahren. Wie im Bereich der Öffnung 24 ist auch im Bereich der Öffnung 66 ein Anschlussstutzen 72 zum Ankoppeln an eine zur Zufuhr bzw. Abfuhr von Behandlungsmedium dienende Anschlussleitung der Reinigungs- und Sterilisationsvorrichtung vorgesehen.

Bei dem in Fig. 5 dargestellten Behälter 2 ist die Öffnung 66 am verjüngten oberen Ende eines auf die Wand 32, 40 des Oberteils 8 aufgesetzten Konus 72 angeordnet, der an seiner Unterseite durch ein gekrümmtes Lochblech 74 verschlossen ist, welches das Eindringen von Stopfen ins Innere des Konus 72 verhindert. Die Form des Lochblechs 74 entspricht der Form einer in der Wand 32, 40 des Oberteils 8 ausgesparten Öffnung am unteren Ende des Konus 72.

Bei dem in Fig. 6 dargestellten Behälter 2 sind die Öffnung 66 und der Anschlussstutzen 72 an einer Stelle in bzw. auf der Außenwand 40 des Behälteroberteils 8 angeordnet, die in Bezug zur Längsachse 38 des Behälterunterteils 4 im Wesentlichen diametral entgegengesetzt zur Befüll- und Entleeröffnung 12 in der Nähe des oberen Randes 36 des Behälterunterteils 4 angeordnet ist. Dadurch wird erreicht, dass die Öffnung 66 einerseits einen möglichst großen Abstand von der Befüll- und Entleeröffnung 12 aufweist und andererseits möglichst hoch über dieser bleibt, um ein Eindringen von Stopfen ins Innere des Anschlussstutzens 72 zu verhindern, wenn der Behälter 2 vor dem Andocken an eine Übergabestation in Richtung des Pfeils A um seine Schwenkachse SA in die Entleerstellung gedreht wird.

Die Befüll- und Entleeröffnung 12 ist wie zuvor durch ein Absperrventil 14 mit Motorantrieb 16 verschließbar, ist jedoch zusätzlich außerhalb des Absperrventils 14 mit einem Beta-Teil 76 eines von der Fa. SNE La Calhene, Frankreich, unter der Bezeichnung DPTE-System vertriebenen Rapid-Transfer-Ports versehen, der mit dem Rand der Befüll- und Entleeröffnung 12 verschraubt ist.

Dieser Beta-Teil 76 des Rapid-Transfer-Ports besteht aus einer die Behälteröffnung 12 verschließenden Klappe 78, einem die Klappe 78 umgebenden Flansch 80 und einer zwischen der Klappe 78 und dem Flansch 80 angeordneten, nach außen weisenden Ringdichtung 82.

Über dem Beta-Teil 76 des Rapid-Transfer-Ports ist eine abnehmbare Druckkappe 84 vorgesehen, die verhindert, dass sich der Beta-Teil während der Behandlung vom Behälter löst und die ein leichtes Anheben des Beta-Teils nach außen zu gestattet, um die Außenseite der Klappe durch Zufuhr von Prozessdampf bei der Behandlung zu sterilisieren.

Vor dem Entleeren des Behälters 2 an der Übergabestation wird die Druckkappe 84 abgenommen und der Beta-Teil 76 des Rapid-Transfer-Ports an einem an der Übergabestation vorgesehenen Alpha-Teil des Rapid-Transfer-Ports (nicht dargestellt) angedockt, wobei das geschlossene Absperrventil 14 in der Entleerstellung verhindert, dass der von innen her nicht belastbare Beta-Teil 76 durch das Gewicht der Stopfen belastet wird. Der Alpha-Teil des Rapid-Transfer-Ports weist ähnlich wie der Beta-Teil eine verschwenkbare Klappe, einen die Klappe umgebenden Flansch und eine zwischen Flansch und Klappe angeordnete Ringdichtung auf, wobei die Klappe jedoch motorisch oder von Hand verschwenkbar ist. Beim Andocken des in den Behälter 2 integrierten Beta-Teils 76 am Alpha-Teil der Übergabestation werden die einander zugewandten Außenseiten der Klappe 78 und der Klappe des Alpha-Teils radial nach außen zu durch dessen Ringdichtung abgedichtet, die sich dichtend gegen der Rand der Klappe 78 des Beta-Teils 76 anlegt. Die Klappe 78 kann dann zusammen mit der Klappe des Alpha-Teils zur Übergabestation hin aufgeschwenkt werden, so dass die Stopfen ohne die Gefahr einer Kontamination aus der Befüll- und Entleeröffnung 12 durch den Port hindurch fallen können, sobald das Absperrventil 14 geöffnet wird.

Das Absperrventil 14 ist ein Klappenventil mit schwenkbarer Klappe, dessen Schwenkwinkel einstellbar ist, so dass die Stopfen durch teilweises Öffnen der Befüll- und Entleeröffnung 12 in die in die Übergabestation dosiert werden können.

Im Unterschied zu dem in den Figuren 1 bis 3 dargestellten Behälter 2 ist die Ebene E der Befüll- und Entleeröffnung 12 bei dem in Fig. 5 dargestellten Behälter 2 parallel zu der vom oberen Rand 36 des Behälterunterteils 4 aufgespannten Ebene und damit bei aufrechtem Behälter horizontal ausgerichtet, so dass die Mittelachse des Öffnungsflanschs 18 der Befüll- und Entleeröffnung 12 zur Längsachse 38 des Behälterunterteils 4 parallel ist, jedoch versetzt zu dieser angeordnet ist. Beim Verschwenken in die Entleerstellung wird der in Fig. 5 dargestellte Behälter 2 um 140 Grad gedreht, so dass die Ebene E der Befüll- und Entleeröffnung 12 unter einem Winkel von 40 Grad zur Horizontalen ausgerichtet ist.

## Patentansprüche

1. Behandlungs- und Transportbehälter (2) zur Behandlung und zum Transport von kleineren Gegenständen, wie Teilen von Spritzen, Verschlusstopfen von Infusionsflaschen oder Vials für pharmazeutische Zwecke oder dergleichen, insbesondere zur Reinigung und Sterilisation der Gegenstände, mit einem Unterteil (4), der eine Längsachse (38) und einen in Richtung der Längsachse (38) im Wesentlichen gleichbleibenden Innenquerschnitt aufweist, einem an einem Stirnende des Unterteils (4) angebrachten Bodenteil (6), der eine mit einem Absperrorgan (20, 22) versehene verschließbare Öffnung (24) für ein Behandlungsmedium aufweist, sowie einem am entgegengesetzten Stirnende des Unterteils (4) angebrachten verjüngten Oberteil (8), der eine mit einem Absperrorgan (14, 16) versehene verschließbare Befüll- und Entleeröffnung (12) aufweist, die in einer Befüllstellung des Behälters (2) nach oben und in einer Entleerstellung des Behälters (2) nach unten weist, wobei einander gegenüberliegende Wandabschnitte (32) des Oberteils (8) unter einem Öffnungswinkel (α) von weniger als 90 Grad von der Befüll- und Entleeröffnung (12) weg divergieren, und wobei außerhalb des Absperrorgans (14) der Befüll- und Entleeröffnung (12) ein Teil (76) eines Rapid-Transfer-Ports angeordnet ist, der bei einem Andocken der Befüll- und Entleeröffnung (12) an einen korrespondierenden Teil eines Rapid-Transfer-Ports einer Übergabestation einen gas- und flüssigkeitsdichten Einschluss der einander zugewandten Außenseiten der beiden korrespondierenden Teile ermöglicht, **dadurch gekennzeichnet, dass** die Befüll- und Entleeröffnung (12) in einem seitlichen Abstand von der Längsachse (38) des Unterteils (4) angeordnet ist, und dass im Abstand von der Befüll- und Entleeröffnung (12) eine Öffnung (66) für ein Behandlungsmedium in den verjüngten Behälteroberteil (8) integriert ist.

2. Behälter nach Anspruch 1, **dadurch gekennzeichnet, dass** aneinandergrenzende Wandabschnitte des Oberteils (8) und des Unterteils (4) entlang eines Teils des Behälterumfangs einen Übergangswinkel (γ) einschließen, der größer ist als die Differenz zwischen 180 Grad und dem halben Öffnungswinkel (α/2).

3. Behälter nach Anspruch 2, **dadurch gekennzeichnet, dass** der Übergangswinkel (γ) zwischen den aneinandergrenzenden Wandabschnitten des Oberteils (8) und des Unterteils (4) an mindestens einem Punkt entlang des Behälterumfangs mehr als 160 Grad und vorzugsweise 180 Grad beträgt.

4. Behälter nach einem der vorangehenden Ansprüche, **dadurch gekennzeichnet, dass** seine Längsachse (38) eine von einem Rand der Befüll- und Entleeröffnung aufgespannte Ebene (E) unter einem Schnittwinkel (ε) schneidet, der kleiner als 90 Grad und vorzugsweise gleich dem Öffnungswinkel (α) ist.

5. Behälter nach einem der vorangehenden Ansprüche, **dadurch gekennzeichnet, dass** ein Schwenkwinkel (β) des Behälters (2) beim Verschwenken aus der Befüllstellung in die Entleerstellung weniger als 180 Grad beträgt.

6. Behälter nach einem der vorangehenden Ansprüche, **dadurch gekennzeichnet, dass** der Behälter (2) an diametral gegenüberliegenden Seiten mit Einrichtungen (28) zum Aufhängen des Behälters (2) versehen ist, und dass die Befüll- und Entleeröffnung (12) in Richtung der einen Einrichtung (28) versetzt ist.

7. Behälter nach einem der vorangehenden Ansprüche, **dadurch gekennzeichnet, dass** die Öffnung (66) entweder auf einer Längsachse (38) des Behälterunterteils (4) oder an einer Stelle des Behälteroberteils (8) angeordnet ist, die in Bezug zu einer Längsachse (38) des Behälterunterteils (4) im Wesentlichen diametral entgegengesetzt zur Befüll- und Entleeröffnung (12) angeordnet ist.

## Claims

1. Treatment and transport container (2) for treating and transporting relatively small objects, such as parts of syringes, closure stoppers of infusion bottles or vials for pharmaceutical purposes or the like, in particular for cleaning and sterilizing the objects, having a lower part (4), which has a longitudinal axis (38) and an internal cross section that remains substantially the same in the direction of the longitudinal axis (38), having a bottom part (6), which is fitted to an end face of the lower part (4) and has a closable opening (24), provided with a shut-off member (20, 22), for a treatment medium, and also having a tapered upper part (8) which is fitted to the opposite end face of the lower part (4) and has a closable filling and emptying opening (12) which is provided with a shut-off member (14, 16) and is directed upwards in a filling position of the container (2) and downwards in an emptying position of the container (2), wherein opposing wall sections (32) of the upper part (8) diverge at an opening angle (α) of less than 90 degrees away from the filling and emptying opening (12), and wherein a part (76) of a rapid-transfer port is arranged outside the shut-off member (14) of the filling and emptying opening (12), said part (76) enabling, when the filling and emptying opening (12) is docked on a corresponding part of a rapid-transfer port of a transfer station, a gas- and fluid-tight enclosure of the mutually facing outsides of the two corresponding parts, **characterized in that** the filling and emptying opening (12) is arranged at a lateral spacing from the longitudinal axis (38) of the lower part (4), and **in that** an opening (66) for a treatment medium is integrated in the tapered container upper part (8) at a spacing from the filling and emptying opening (12).

2. Container according to Claim 1, **characterized in that** adjoining wall sections of the upper part (8) and of the lower part (4) enclose, along a part of the periphery of the container, a transition angle (γ) which is greater than the difference between 180 degrees and half the opening angle (α/2).

3. Container according to Claim 2, **characterized in that** the transition angle (γ) between the adjoining wall sections of the upper part (8) and of the lower part (4) is more than 160 degrees and preferably 180 degrees at at least one point along the periphery of the container.

4. Container according to one of the preceding claims, **characterized in that** its longitudinal axis (38) intersects a plane (E) spanned by an edge of the filling and emptying opening at an intersection angle (ε) which is less than 90 degrees and preferably the same as the opening angle (α).

5. Container according to one of the preceding claims, **characterized in that** a pivot angle (β) of the container (2) when pivoting out of the filling position and into the emptying position is less than 180 degrees.

6. Container according to one of the preceding claims, **characterized in that** the container (2) is provided at diametrically opposite sides with devices (28) for suspending the container (2), and **in that** the filling and emptying opening (12) is offset in the direction of one device (28).

7. Container according to one of the preceding claims, **characterized in that** the opening (66) is arranged either on a longitudinal axis (38) of the container lower part (4) or at a point of the container upper part (8) which, in relation to a longitudinal axis (38) of the container lower part (4), is arranged substantially diametrically opposite the filling and emptying opening (12).

## Revendications

1. Conteneur de traitement et de transport (2) pour le traitement et le transport d'objets relativement petits, tels que des parties de seringues, des bouchons de fermeture de bouteilles d'infusion ou de flacons à usage pharmaceutique ou similaires, en particulier pour le nettoyage et la stérilisation des objets, comprenant une partie inférieure (4), qui présente un axe longitudinal (38) et une section transversale intérieure essentiellement uniforme dans la direction de l'axe longitudinal (38), une partie de fond (6) montée sur une extrémité frontale de la partie inférieure (4), qui présente une ouverture (24) refermable pourvue d'un organe d'arrêt (20, 22) pour un fluide de traitement, ainsi qu'une partie supérieure (8) rétrécie montée sur l'extrémité frontale opposée de la partie inférieure (4), qui présente une ouverture de remplissage et de vidange (12) refermable pourvue d'un organe d'arrêt (14, 16), qui est tournée vers le haut dans une position de remplissage du conteneur (2) et vers le bas dans une position de vidange du conteneur (2), des portions de paroi mutuellement opposées (32) de la partie supérieure (8) divergeant d'un angle d'ouverture (α) inférieur à 90 degrés à l'écart de l'ouverture de remplissage et de vidange (12), et à l'extérieur de l'organe d'arrêt (14) de l'ouverture de remplissage et de vidange (12) étant disposée une partie (76) d'un orifice de transfert rapide, laquelle permet, lors d'un arrimage de l'ouverture de remplissage et de vidange (12) à une partie correspondante d'un orifice de transfert rapide d'un poste de transfert, un raccord étanche aux gaz et aux liquides des côtés extérieurs tournés l'un vers l'autre des deux parties correspondantes, **caractérisé en ce que** l'ouverture de remplissage et de vidange (12) est disposée à distance latérale de l'axe longitudinal (38) de la partie inférieure (4), et **en ce qu'**une ouverture (66) pour un fluide de traitement est intégrée dans la partie supérieure rétrécie du conteneur (8) à distance de l'ouverture de remplissage et de vidange (12).

2. Conteneur selon la revendication 1, **caractérisé en ce que** des portions de paroi adjacentes l'une à l'autre de la partie supérieure (8) et de la partie inférieure (4) forment le long d'une partie de la périphérie du conteneur un angle de transition (γ) qui est supérieur à la différence entre 180 degrés et le demi-angle d'ouverture (α/2).

3. Conteneur selon la revendication 2, **caractérisé en ce que** l'angle de transition (γ) entre les portions de paroi adjacentes l'une à l'autre de la partie supérieure (8) et de la partie inférieure (4) vaut plus de 160 degrés et de préférence 180 degrés au niveau d'au moins un point le long de la périphérie du conteneur.

4. Conteneur selon l'une quelconque des revendications précédentes, **caractérisé en ce que** son axe longitudinal (38) coupe un plan (E) tendu par un bord de l'ouverture de remplissage et de vidange en formant un angle de coupe (ε) qui est inférieur à 90 degrés et de préférence égal à l'angle d'ouverture (α).

5. Conteneur selon l'une quelconque des revendications précédentes, **caractérisé en ce qu'**un angle de pivotement (β) du conteneur (2) lors du pivotement de la position de remplissage dans la position de vidange est inférieur à 180 degrés.

6. Conteneur selon l'une quelconque des revendications précédentes, **caractérisé en ce que** le conteneur (2) est pourvu sur des côtés diamétralement opposés de dispositifs (28) pour accrocher le conteneur (2), et **en ce que** l'ouverture de remplissage et de vidange (12) est décalée dans la direction de l'un des dispositifs (28).

7. Conteneur selon l'une quelconque des revendications précédentes, **caractérisé en ce que** l'ouverture (66) n'est disposée ni sur un axe longitudinal (38) de la partie inférieure du conteneur (4) ni en un emplacement de la partie supérieure du conteneur (8) qui est disposé par rapport à un axe longitudinal (38) de la partie inférieure du conteneur (4) essentiellement de manière diamétralement opposée à l'ouverture de remplissage et de vidange (12).
